# EUROPÄISCHE PATENTSCHRIFT

(11) **EP 1 235 601 B1**
(45) Veröffentlichungstag und Bekanntmachung des Hinweises auf die Patenterteilung: **26.07.2006**
(21) Anmeldenummer: 00979676.4
(22) Anmeldetag: 08.12.2000
(51) Int. Cl.: A61M 1/16

(54) **Dispoable für eine Vorrichtung zur Durchführung einer medizinischen Behandlung unter Verwendung von Flüssigkeiten**
Disposable element for a medical treatment device using a liquid
Objet à usage unique pour un dispositif de traitement médical utilisant un liquide

(30) Priorität: 08.12.1999 DE 19959230
(43) Veröffentlichungstag der Anmeldung: 04.09.2002
(60) Teilanmeldung: 06005573.8
(73) Patentinhaber: Fresenius Medical Care Deutschland GmbH, 61352 Bad Homburg v.d.H. (DE)
(72) Erfinder: HILGERS, Peter, 97453 Schonungen (DE); BRANDL, Matthias, 97631 Bad Königshofen (DE)
(74) Vertreter: Luderschmidt, Schüler & Partner GbR
(86) Internationale Anmeldenummer: PCT/EP2000/012417
(87) Internationale Veröffentlichungsnummer: WO 2001/041834

(56) Entgegenhaltungen:
- FR-A- 2 749 763
- US-A- 5 364 385
- US-A- 5 607 082

## Beschreibung

Die Erfindung betrifft ein Disposable für eine Vorrichtung zur Durchführung einer medizinischen Behandlung unter Verwendung einer Flüssigkeit. Darüber hinaus bezieht sich die Erfindung auf die Verwendung des Disposables in einer Hämodialysevorrichtung.

Hämodialysegeräte sind in verschiedenen Ausführungen bekannt. Der Stoffaustausch zwischen dem Blut und der Dialysierflüssigkeit findet in einem Dialysator statt, der einen ersten Strömungsweg für das Blut und einen zweiten Strömungsweg für die Dialysierflüssigkeit aufweist, wobei beide Strömungswege durch eine semipermeable Membran voneinander getrennt sind. Der erste Strömungsweg ist Bestandteil eines extrakorporalen Blutkreislaufs mit einer Zuführleitung und einer Rückführleitung für das Blut sowie ggf. einer den Blutfluß unterstützenden Pumpe. Der zweite Strömungsweg ist mit Einrichtungen zum Zuführen und Abführen der Dialysierflüssigkeit verbunden. Neben den sogenannten Single-Pass-Systemen, bei denen die kontinuierlich zugeführte Dialysierflüssigkeit nur einmal den Dialysator passiert und dann verworfen wird, sind auch sogenannte Batch-Systeme bekannt. Die DE 31 15 665 C2 beschreibt ein derartiges Hämodialysiergerät, das mit einem gegen die Atmosphäre abgeschlossenen, volumenstarren Behälter arbeitet, der vor Beginn der Behandlung vollständig mit frischer Dialysierflüssigkeit gefüllt wird. Während des Betriebs wird Flüssigkeit aus dem Behälter durch den Dialysator gepumpt und die verbrauchte Flüssigkeit wird wieder in den Behälter zurückgeleitet. Aufgrund des konstanten Volumens des gesamten mit der Dialysierflüssigkeit gefüllten Systems kann eine Ultrafiltration nur dann erfolgen, wenn dem System Flüssigkeit entzogen wird. Eine Vermischung von frischer und verbrauchter Dialysierflüssigkeit wird bei dem bekannten Hämodialysegerät dadurch vermieden, daß die Entnahme der Dialysierflüssigkeit im oberen Bereich des Behälters erfolgt, während die Rückführung im unteren Behälterbereich stattfindet. Die Unterschichtung der frischen Dialysierflüssigkeit mit der verbrauchten Dialysierflüssigkeit bleibt durch die Aufrechterhaltung eines vertikalen Temperaturgefälles in dem Behälter von oben nach unten stabil.

Der Behälter besteht aus Glas, das weitgehend resistent gegen in Betracht kommende Chemikalien, gut zu reinigen und physiologisch unbedenklich ist. Nachteilig ist jedoch, daß der Glasbehälter verhältnismäßig teuer in der Herstellung und relativ schwierig zu reinigen ist.

Aus der DE 198 25 158 C1 ist ein als Folienbeutel ausgebildetes Disposable bekannt, daß zusammen mit einer formgebenden Schale den in der Herstellung relativ teueren Glasbehälter der Hämodialysevorrichtung ersetzen kann, die in der eingangs erwähnten DE 31 15 665 C2 beschrieben ist. Das Disposable besteht aus zwei drehkegelförmigen Teilstücken, die an ihrem umlaufenden Rand zu einem Doppelkegel miteinander verschweißt sind. Der besondere Vorteil dieser Formgebung liegt daran, daß sich das Disposable aus handelsüblichen (ebenen) Folien herstellen läßt. Eine räumliche Formgebung, wie beispielsweise bei Einmal-Handschuhen, ist hierzu nicht erforderlich.

Der Glasbehälter kann nur dann durch den Folienbeutel ersetzt werden, wenn die Volumenkonstanz, Keimfreiheit und Flüssigkeitsunterschichtung gewährleistet ist.

Um die Volumenkonstanz sicherzustellen, muß sich der Beutel während des Füllens reproduzierbar und vollständig in der druckstabilen Schale mit definiertem Volumen entfalten können. Lufteinschlüsse zwischen Beutel und Schale, die durch Entfaltungen oder zu überbrückende Zwischenräume entstehen, müssen weitgehend vermieden werden. Durch die Geometrie bedingte Falten können nur dann akzeptiert werden, sofern sie reproduzierbar sind und das von ihnen eingeschlossene Volumen im Vergleich zum Gesamtvolumen vernachlässigbar ist. Zur Gewährleistung der Keimfreiheit darf der Innenraum des Beutels während der Dialysevorbereitung nicht geöffnet und während des Füllens nur in Kontakt mit der einströmenden Flüssigkeit kommen. Zur Flüssigkeitsunterschichtung muß die Form des Beutels eine klare Trennung zwischen frischem und verbrauchtem Dialysat unterstützen. Darüber hinaus soll der Beutel einfach zu handhaben und kostengünstig herzustellen sein.

Die FR 2 749 763 beschreibt ein Verfahren zur Herstellung eines Salzkonzentrates für die Hämodialyse. Das Salzkonzentrat wird in einem Beutel bereitgestellt, der aus einer Schlauchfolie gefaltet wird. Es handelt sich um einen Seitenfaltenbeutel, der sich zu einer rechteckförmigen Packung aufklappen läßt.

Die US-A-5 607 082 beschreibt einen Tank für eine Sprühpistole mit einem Außenbehälter, der einen Innenbehälter aufnimmt. Der Innenbehälter weist eine feste Rückwand auf und ist harmonikaartig gefaltet.

Aus der DE 9417416 U1 ist ein Sekretauffangbeutel bekannt, der harmonikaartig zusammenfaltbar ist.

Der Erfindung liegt die Aufgabe zugrunde, ein einfach zu handhabendes und kostengünstig herstellbares Disposable für eine medizinische Behandlungsvorrichtung zu schaffen, das die erforderliche Volumenkonstanz, Keimfreiheit und Flüssigkeitsunterschichtung gewährleistet.

Die Lösung dieser Aufgabe erfolgt mit dem Gegenstand des Patentanspruchs 1.

Das erfindungsgemäße Disposable wird aus zwei übereinanderliegenden Flachfolien oder einer Schlauchfolie hergestellt, wobei die ein oder zwei Längsschweißnähte zum Versiegeln der übereinanderliegenden Flachfolien bei der Verwendung einer Schlauchfolie entfallen können.

An dem in der Gebrauchslage oberen Ende ist der Folienbeutel bzw. die Schlauchfolie mit einer ersten Schweißnaht und an dem unteren Ende mit einer zweiten Schweißnaht verschlossen. Beide Schweißnähte haben vorzugsweise einen nach außen gekrümmten Verlauf. Der entfaltete Folienbeutel hat damit vorzugsweise einen zylindrischen Abschnitt, an den sich ein nach außen gewölbter oberer und ein nach außen gewölbter unterer Abschnitt anschließen. Das gleiche gilt für die Schlauchfolie.

Unter nach außen gekrümmten Schweißnähten sind aber auch Schweißnähte zu verstehen, die sich aus mehreren geraden Abschnitten zusammensetzten. So hat es sich in der Praxis als besonders vorteilhaft erwiesen, anstelle von bogenförmigen Schweißnähten Schweißnähte mit geraden Abschnitten vorzusehen, die den Verlauf der Bogenschweißnähte approximieren. Derartige Schweißnähte lassen sich produktionstechnisch besonders einfach anfertigen.

Eine medizinische Behandlungsvorrichtung für das erfindungsgemäße Disposable verfügt über eine als formgebende Schale ausgebildete Aufnahmeeinheit für das Disposable. Die formgebende Schale weist eine mittlere zylindrische Anlagefläche auf, an die sich eine nach außen gewölbte obere Anlagefläche und eine nach außen gewölbte untere Anlagefläche anschließen. Die Schale zeichnet sich somit durch eine hohe Druckstabilität aus.

Für die medizinische Behandlung wird das erfindungsgemäße Disposable in die formgebende Schale einer Behandlungsvorrichtung eingelegt. Da sich die gewölbten Abschlüsse des Zylinders durch ebene Flächen nicht exakt abwickeln lassen, ist zwar eine Faltenbildung des Folienbeutels bzw. der Schlauchfolie in den Abschlüssen unvermeidbar, in der Praxis hat sich jedoch gezeigt, daß das von den Falten in den Abschlüssen eingeschlossene Volumen im Vergleich zum Gesamtvolumen relativ gering ist.

Der Folienbeutel bzw. die Schlauchfolie ist parallel der in der Gebrauchslage vertikalen Längsachse mehrfach harmonikaartig gefaltet. Vorzugsweise ist der Folienbeutel bzw. die Schlauchfolie mehrfach in gleich große Abschnitte gefaltet, wobei es für einen symmetrischen Aufbau vorteilhaft ist, eine ungerade Anzahl von Faltabschnitten zu wählen.

Der gefaltete Folienbeutel bzw. die Schlauchfolie bildet einen flachen Streifen.

Das Disposable stellt eine aufgrund der einfachen geometrischen Form kostengünstig herzustellende Anordnung dar, die sich einfach handhaben läßt. Die Beutelform unterstützt die reproduzierbare Entfaltung. Neben der Optimierung des Ausbreitungsverhaltens hat die Faltung auch den Vorteil, daß der Beutel bei der Lagerung und dem Transport weniger Raum einnimmt.

Um das Disposable als flachen Streifen bereitstellen zu können, sind die Faltungen mit Verbindungselementen, beispielsweise Nieten, Klammern oder dgl. fixiert, die an den Folienenden angeordnet sind. Es können aber auch Schweißnähte senkrecht quer zur Längsachse vorgesehen sein.

In einer bevorzugten Ausführungsform des Disposables ist an der ersten Schweißnaht ein Anschlußteil mit der Beutelfolie verschweißt, der mindestens einen Anschluß zum Zuführen von Flüssigkeit und einen Anschluß zum Abführen von Flüssigkeit aufweist. An dem Anschluß zum Zuführen von Flüssigkeit ist eine sich in das Beutelinnere bis zu der zweiten Schweißnaht erstreckende Schlauchleitung angeschlossen. Dadurch wird erreicht, daß Flüssigkeit, beispielsweise verbrauchte Dialysierflüssigkeit, der unteren Hälfte zugeführt und aus der oberen Hälfte des Disposables Flüssigkeit, beispielsweise frische Dialysierflüssigkeit, abgezogen werden kann.

Zur Fixierung der Schlauchleitung in dem Disposable kann an der zweiten Schweißnaht ein Fixierungsstück vorgesehen sein, an dem die Schlauchleitung befestigt ist.

Der Folienbeutel bzw. die Schlauchfolie besteht vorzugsweise aus einem Polyethylen-Basismaterial, das einseitig mit einer Polyamid-Siegelschicht versehen ist.

Die Faltungen geben eine parallel und eine senkrecht zur Flächennormalen der Faltenabschnitte gerichtete Orientierung vor. Diese Orientierungen sind durch die Mehrlagigkeit der Faltenabschnitte versteift, wodurch sie beim Einführen des Disposables in die formgebende Schale einer Behandlungsvorrichtung raumfest erhalten bleiben.

Der Verlauf der ersten und zweiten Schweißnaht wird vorzugsweise so gewählt, daß die Faltenbildung in den Zylinderabschlüssen minimiert und reproduzierbar ist. Dies kann insbesondere mit einer bogenförmigen Schweißnaht erreicht werden, die nur Falten senkrecht zur Naht erzeugt. Wenn das Disposable befüllt ist, sind bei einer bogenförmigen Schweißnaht die auftretenden Spannungen verhältnismäßig gering.

In Versuchen hat sich gezeigt, daß sich das Disposable insbesondere dann leicht entfalten und an die Anlageflächen der Schale anlegen kann, wenn der Abstand zwischen den Enden der ersten und zweiten Schweißnaht auf einer der beiden Längsseiten des flachliegenden Folienbeutels bzw. der Schlauchfolie größer ist, als die Länge der zylindrischen Anlagefläche der formgebenden Schale. Der Abstand sollte vorzugsweise um einen Betrag größer sein, der mindestens doppelt so groß, vorzugsweise dreimal so groß wie die Breite der Falten des Folienbeutels bzw. der Schlauchfolie ist.

Eine weitere Verbesserung wird dadurch erzielt, wenn gleichzeitig der Abstand zwischen dem Scheitelpunkt der ersten bzw. zweiten Schweißnaht und dem Mittelpunkt der durch deren Enden verlaufenden Geraden bei flachliegendem Folienbeutel bzw. Schlauchfolie kleiner als die Tiefe der gewölbten Anlagefläche der formgebenden Schale ist. Damit wird die Krümmung der Bogenschweißnähte geringer, wodurch sich diese in ihren Randbereichen auch durch gerade Schweißnähte ersetzen lassen.

Das Disposable findet vorzugsweise in einer Hämodialysevorrichtung Verwendung. Es kann aber auch ähnlich wie in der DE 198 25 158 C1 beschrieben in einer Peritonealdialyse-Vorrichtung zur Bereitstellung von Peritonealdialyse-Lösung verwendet werden.

Im folgenden werden einzelne Ausführungsbeispiele der Erfindung unter Bezugnahme auf die Zeichnungen näher erläutert.

Es zeigen:
- Figur 1: eine vereinfachte Darstellung des flachliegenden erfindungsgemäßen Disposables in der Draufsicht,
- Figur 2: das gefaltete Disposable von Figur 1,
- Figur 3: die harmonikaartige Faltung des Disposables,
- Figur 4: eine vereinfachte Darstellung einer Aufnahmeeinheit für das erfindungsgemäße Disposable von Figur 1,
- Figur 5: den Ausschnitt A von Figur 4 in vergrößerter perspektivischer Darstellung,
- Figur 6: den Ausschnitt B von Figur 4 in vergrößerter perspektivischer Darstellung,
- Figur 7: die Aufnahmeeinheit von Figur 4, in die das Disposable von Figur 1 eingelegt ist.
- Figur 8: die an die Aufnahmeeinheit angepaßten Schweißnähte des Disposables von Figur 1,
- Figur 9: eine Hämodialysevorrichtung mit der Aufnahmeeinheit von Figur 4 und dem Disposable von Figur 1 in vereinfachter Darstellung und
- Figur 10: ein weiteres Ausführungsbeispiel des Disposables in der Draufsicht.

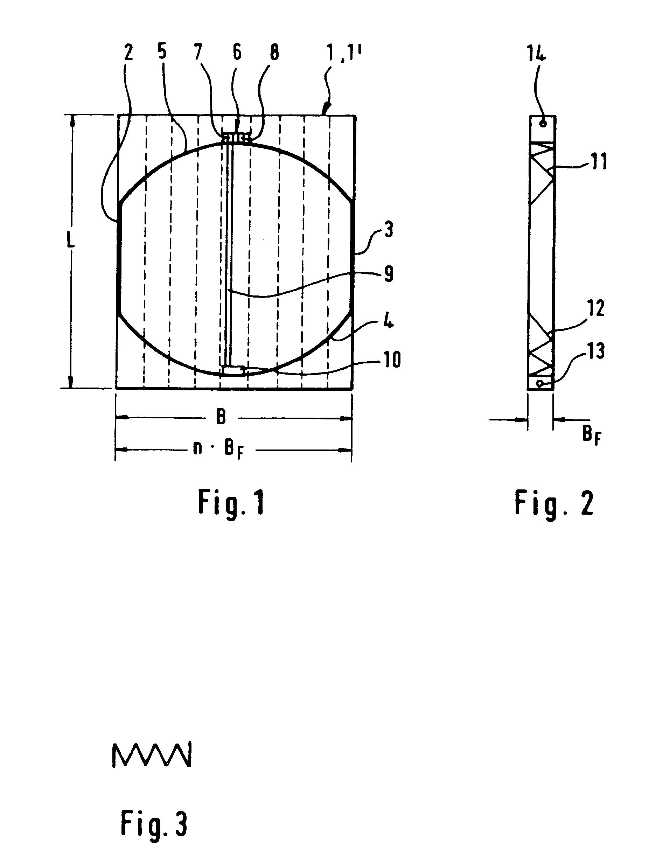

Figur 1 zeigt die Draufsicht auf das flachliegende Disposable in vereinfachter Darstellung. Das Disposable weist zwei rechteckförmige Folien 1, 1' mit der Länge L und der Breite B auf, die deckungsgleich übereinander liegen und an den Längsseiten mit Längsschweißnähten 2, 3 und an den Enden mit nach außen gekrümmten Bogenschweißnähten 4,5 versiegelt sind. Als Folienmaterial wird eine Flachfolie verwendet, die aus Polyethylen (PE) als Basismaterial besteht, das einseitig mit einer Polyamid (PA) -Siegelschicht versehen ist. Die Dicke der Folie beträgt 100 µm.

Anstelle der übereinander liegenden Flachfolien kann auch ein Folienschlauch verwendet werden, der an seinen Enden mit den Bogenschweißnähten verschlossen wird. Es ist aber auch möglich eine Flachfolie zu verwenden, die einseitig gefaltet, nur an einer Längsseite mit einer Längssschweißnaht versiegelt und an ihren Enden mit den Bogenschweißnähten verschlossen wird.

Das Disposable verfügt über einen in Figur 1 nur andeutungsweise dargestellten Anschlußteil 6, der beispielsweise nach Art eines Schiffchens ausgebildet und mit den Flachfolien versiegelt ist. Der Anschlußteil 6 weist einen Anschluß 7 zum Zuführen und einen Anschluß 8 zum Abführen von Flüssigkeit auf. An den Anschlüssen 7 und 8 werden die Schlauchleitungen der medizinischen Behandlungsvorrichtung angeschlossen.

Der Anschlußteil 6 ist am Scheitelpunkt der in der Gebrauchslage des Disposables oberen Bogenschweißnaht 5 angeordnet. An dem Anschluß 7 zum Zuführen von Flüssigkeit ist eine sich in das Beutelinnere bis zu der unteren Bogenschweißnaht 4 erstreckende Schlauchleitung 9 angeschlossen, deren freies Ende an einem Fixierungsstück 10 befestigt ist, das am Scheitelpunkt der unteren Schweißnaht 4 mit den Flachfolien 1, 1' versiegelt ist. Der flachliegende Folienbeutel wird parallel zu seiner Längsachse mehrfach in gleich große Abschnitte harmonikaartig gefaltet. Die Breite B_{F} der Faltabschnitte berechnet sich aus dem Quotienten der Breite B der Flachfolien und der Anzahl n der Faltabschnitte. Für einen symmetrischen Aufbau ist es zweckmäßig, eine ungerade Anzahl von Faltabschnitten zu wählen (z. B. n = 9).

Figur 2 zeigt den harmonikaartig gefalteten Folienbeutel von Figur 1 in der Draufsicht. Die gefalteten Bogenschweißnähte an den Enden des Folienbeutels sind mit den Bezugszeichen 11, 12 versehen. Figur 3 zeigt die harmonikaartige Faltung des Folienbeutels.

Zur Fixierung der Faltungen sind in Figur 2 nur andeutungsweise dargestellte Verbindungselemente 13, 14 vorgesehen, die an den über die Bogenschweißnähte 4, 5 überstehenden Enden des Folienbeutels angeordnet sind. Die Verbindungselemente sind Nieten, mit denen der Folienstapel als flacher Streifen fest zusammengehalten wird.

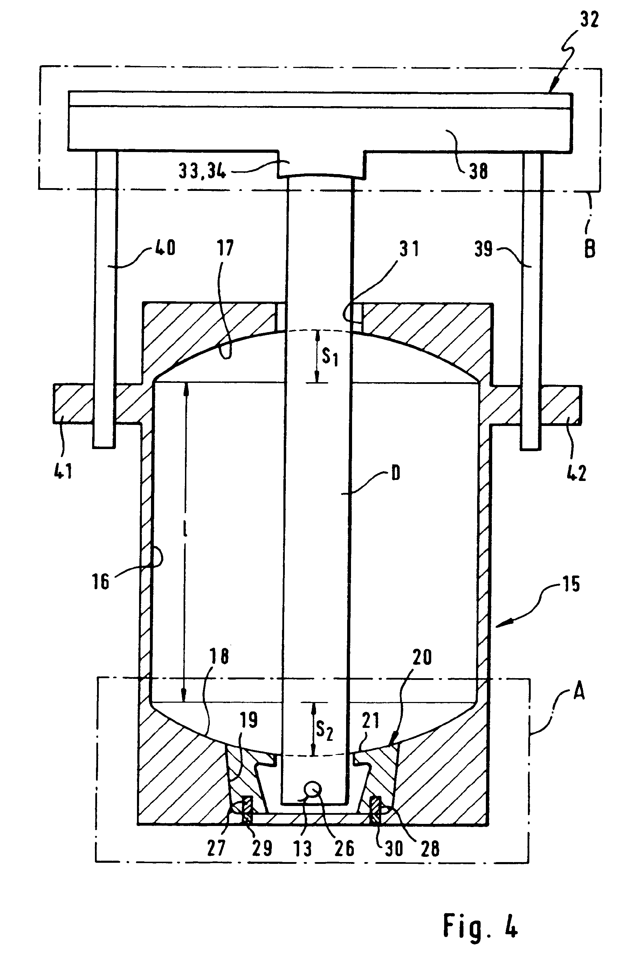

Nachfolgend wird unter Bezugnahme auf die Figuren 4 bis 7 eine Aufnahmeeinheit für das erfindungsgemäße Disposable beschrieben, die Bestandteil der medizinischen Behandlungsvorrichtung, beispielsweise einer Hämodialysevorrichtung ist.

Die Aufnahmeeinheit ist als formgebende Schale ausgebildet, in der das Disposable nach der Entfaltung eine genau reproduzierbare Form erhält. Die formgebende Schale 15 weist eine mittlere zylindrische Anlagefläche 16 auf, an die sich als Deckel eine nach außen gewölbte obere Anlagefläche 17 und als Boden eine nach außen gewölbte untere Anlagefläche 18 anschließt. Insofern bildet die formgebende Schale einen rotationssymmetrischen Hohlkörper, der das Disposable vollständig aufnimmt. Die obere und untere Anlagefläche 17, 18 weisen im Schnitt eine bogenförmige Kontur auf. Die Abstände zwischen den Scheitelpunkten der bogenförmigen Konturen der oberen bzw. unteren Anlagefläche 17, 18 und der zylindrischen Anlagefläche 16, d. h. die Höhe bzw. Tiefe der nach außen gewölbten Abschnitte, sind in Figur 4 mit S₁ und S₂ bezeichnet. Der zylindrische Abschnitt 16 hat die Länge 1.

Die Anlageflächen 17 und 18 können eine gleiche oder unterschiedlichen Wölbung aufweisen, wobei dies ebenfalls für die geometrische Ausgestaltung des Disposables gültig ist. So kann es zweckmäßig sein, die obere gewölbte Fläche 17 tiefer anzuordnen, damit ein möglichst eindeutig definierter Punkt für die Dialsysierflüssigkeitsentnahme vorliegt. Weiterhin kann es vorteilhaft sein, wenn die untere gewölbte Fläche 18 zur Minimierung der Bauhöhe eine flache Wölbung aufweist.

Die Schale besteht vorzugsweise aus einem wärmeisolierendem Material oder ist mit einer Wärmeisolierung versehen.

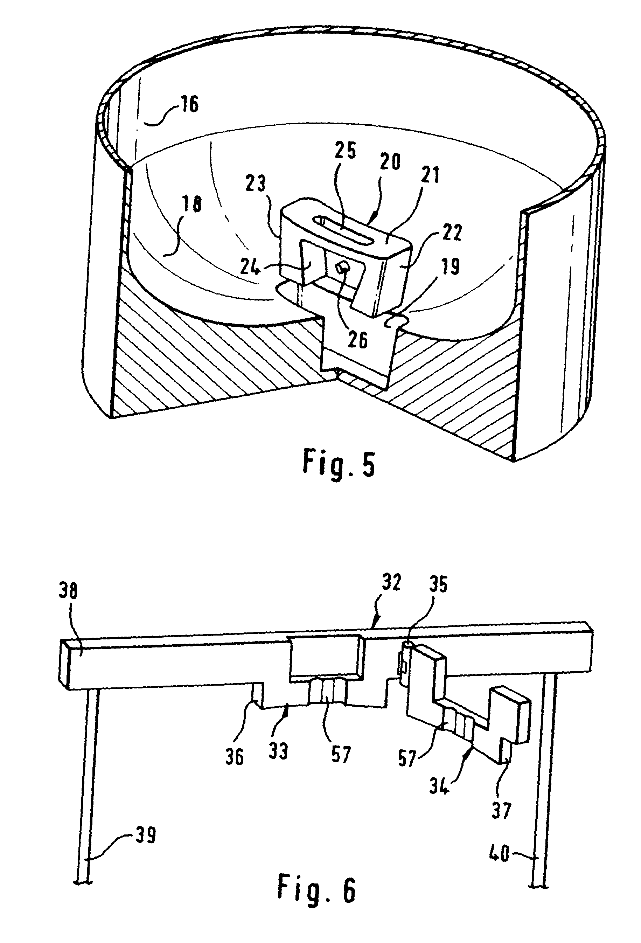

Im Zentrum der unteren Anlagefläche weist die Schale 15 eine Ausnehmung 19 auf, in die eine Fixierungseinrichtung 20 zur Befestigung des unteren Endes des Disposables D paßgenau einsetzbar ist. Die Fixierungseinrichtung 20 ist ein längliches Formstück mit einer der Kontur der unteren Anlagefläche 18 entsprechenden Oberseite 21 und zwei nach innen verlaufenden Schmalseiten 22, 23, so daß sich das Formstück beim Einsetzen in die Schale zentriert (Figur 5).

Eine der Längsseiten des Formstücks ist mit einer mittigen Ausparung 24 versehen. An seiner Oberseite weist das Formstück ein Langloch 25 auf, durch das sich das untere Ende des gefalteten Disposables D in das Formstück einschieben läßt. Zur Befestigung des Disposables befindet sich in der Aussparung 24 unterhalb des Langlochs 25 ein Stift 26. Die Fixierung des Formstücks erfolgt mittels einer Magnetkupplung, die aus zwei in das Formstück eingesetzten Magneten 27, 28 und zwei in die Schale 15 eingesetzten Magneten 29, 30 besteht. Die Fixierung kann leicht durch eine nicht näher dargestellt Mimik gelöst werden, bei der die Magneten 29, 30 über ein Fußpedal von den Magneten 27, 28 gelöst werden. Anstelle einer Magnetkupplung können aber auch andere Ver- und Entriegelungseinrichtungen vorgesehen, die manuell oder automatisch betätigt werden können.

Die Schale 15 weist im Zentrum der oberen Anlagefläche 17 eine weitere Ausnehmung 31 auf. Die Ausnehmung 31 ist ein Schlitz, der derart bemessen ist, daß die Fixierungseinrichtung 20 von oben in die Schale eingeführt werden kann. Zur Befestigung des oberen Endes des Disposables und zum Verschließen des Schlitzes ist eine weitere Fixierungseinrichtung 32 vorgesehen.

Figur 6 zeigt die Fixierungseinrichtung 32 in perspektivischer Darstellung. Die Fixierungseinrichtung 32 weist zwei Formstücke 33, 34 auf, wobei das eine Formstück 34 mit einem Scharnier 35, klappbar an dem anderen Formstück 33 befestigt ist. Beide Formstücke 33, 34 verfügen über einen unteren Abschnitt 36, 37, die paßgenau in den Schlitz 31 einsetzbar sind, wenn die Formstücke zusammengeklappt sind. Das Formstück 36 ist einstückig mit einem horizontalen Balken 38, an dem vertikale Führungsstangen 39, 40 befestigt sind, die in seitlichen Führungsstücken 41, 42 der Schale 15 in Längsrichtung verschiebbar geführt sind.

Die unteren Abschnitte 36, 37 der Formstücke 33, 34 weisen eine Kontur auf, die der Kontur der oberen Anlagefläche 17 entspricht. An den Innenseiten der beiden Formstücke 33, 34 ist eine muldenförmige Vertiefung 57 zur Aufnahme des Anschlußteils 6 des Disposables vorgesehen.

Für die medizinische Behandlung wird das untere Ende des gefalteten Disposables D durch die Ausnehmung 25 der unteren Fixierungseinrichtung 20 geschoben und das Disposable wird mittels der eine Öse bildenden Niete 13 an dem Stift 26 aufgehängt. Dann wird die untere Fixierungseinrichtung 20 an dem Disposable heruntergelassen, bis sie die untere Ausnehmung 19 der Schale verschließt. Damit ist das untere Ende des Disposables fixiert. Daraufhin wird der obere Anschlußteil 6 des Disposables in die muldenförmige Vertiefung 57 der oberen Fixierungseinrichtung 32 eingelegt und durch Zusammenklappen der Formstücke 33, 34 verklemmt. Damit ist auch das obere Ende des Disposables fixiert (Figur 4).

Beim Befüllen wird die zwischen Folienbeutel und Schale 15 befindliche Luft über eine nicht näher dargestellte Belüftungsöffnung in der Schale herausgedrückt.

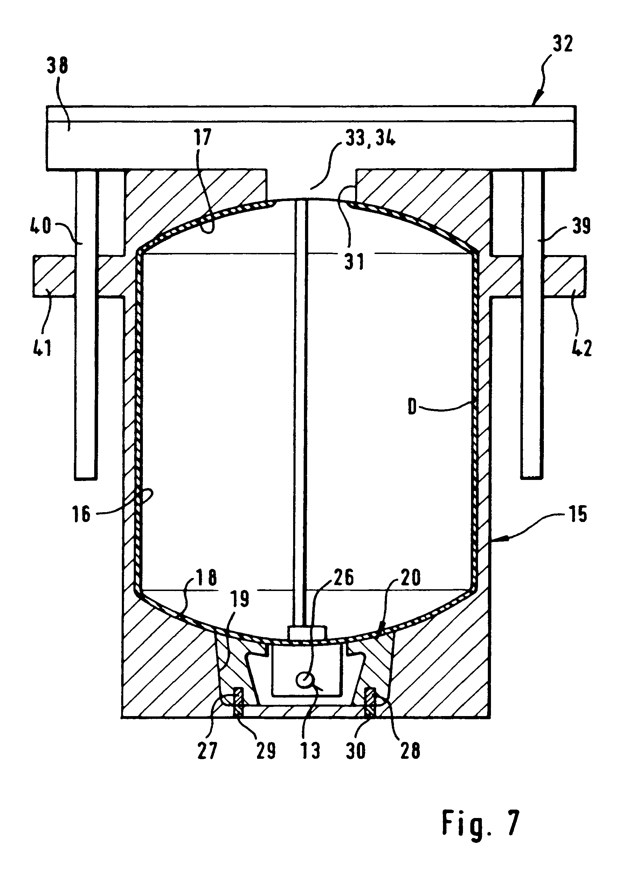

Beim Befüllen des Disposables mit Flüssigkeit entfaltet sich der Folienbeutel in der Schale. Wenn sich der Folienbeutel in der Schale entfaltet, verschiebt sich die obere Fixierungseinrichtung 32 aufgrund der Verkürzung des Folienbeutels, bis sie die obere Ausnehmung 31 vollständig verschließt (Figur 7). Der zylindrische Abschnitt des befüllten Folienbeutels liegt nun faltenfrei an dem zylindrischen Abschnitt der Schale an. Auch die nach außen gewölbten oberen und unteren Abschnitte des Folienbeutels liegen weitgehend faltenfrei an den oberen und unteren Anlageflächen der Schale an, so daß eine genau reproduzierbare Form gegeben ist.

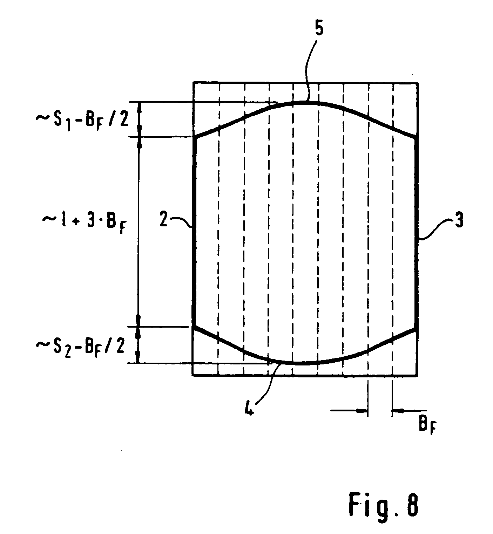

Figur 8 zeigt ein Disposable, das sich besonders einfach entfalten läßt und in seinen oberen und unteren Abschnitten zu einer besonders geringen Faltenbildung neigt. Die Bezugszeichen entsprechen den Bezugszeichen des unter Bezugnahme auf die Figuren 1 bis 3 beschriebenen Disposables. Die Länge der beiden Längsschweißnähte 2, 3 des flachliegenden Folienbeutels ist größer als die Länge 1 des zylindrischen Abschnitts der Schale 15. Sie ist um einen Betrag größer, der etwa mindestens doppelt so groß wie die Breite B_{F} der Falten des Folienbeutels ist. Eine an die Schale optimale Anpassung wird dann erreicht, wenn die Länge der Längsschweißnähte 2, 3 um einen Betrag größer ist, der etwa dreimal so groß wie die Faltenbreite B_{F} ist. Der Abstand zwischen dem Mittelpunkt der durch die gegenüberliegenden Enden der Bogenschweißnähte 4, 5 verlaufenden Geraden und den Scheitelpunkten der Bogenschweißnähte ist geringer als die Höhe bzw. Tiefe S₁, S₂ der nach außen gewölbten Abschnitte 17, 18 der Schale 15. Als optimal hat sich erwiesen, wenn der Abstand zwischen den Scheitelpunkten und den Mittelpunkten der Geraden um etwa die Hälfte der Faltenbreite B_{F} kleiner ist. Damit erhält die Bogenform einen relativ flachen Verlauf, so daß deren auslaufende Enden auch durch gerade Schweißnähte ersetzt werden können.

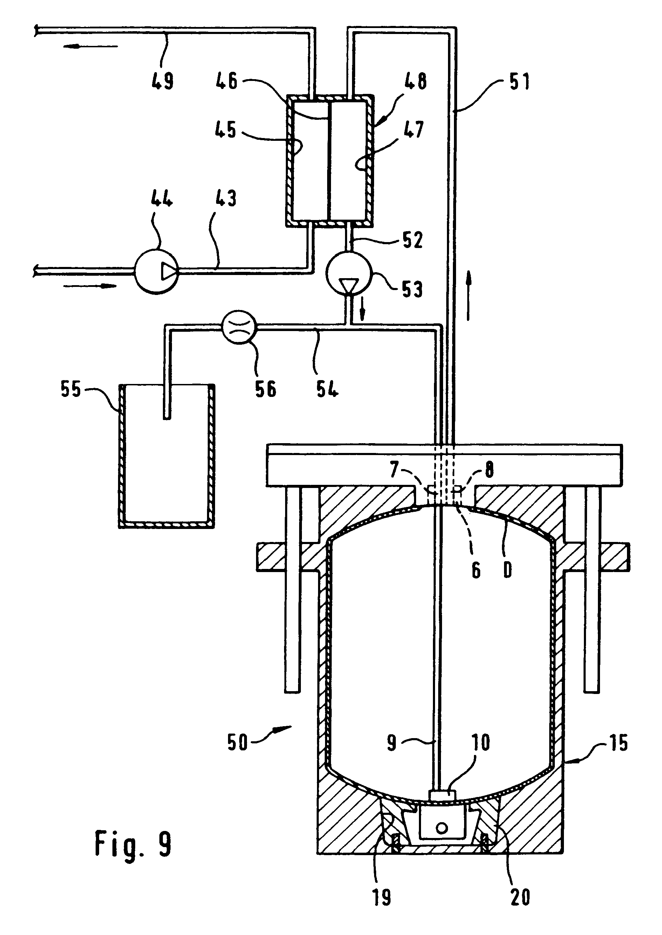

Im folgenden wird unter Bezugnahme auf Figur 9 eine Hämodialysevorrichtung beschrieben, die eine Aufnahmeeinheit gemäß der Figuren 4 bis 7 aufweist und bei der ein Disposable gemäß der Figuren 1 bis 3 bzw. 8 verwendet wird. Die Bezugszeichen von Figur 8 entsprechen den Bezugszeichen der Figuren 1 bis 8.

Das Blut des Patienten wird über eine arterielle Blutleitung 43, in die eine Blutpumpe 44 geschaltet ist, der Blutkammer 45 eines durch eine semipermeable Membran 46 in zwei Kammern 45, 47 unterteilten Dialysators 48 zugeführt und über eine venöse Blutleitung 49 zum Patient zurückgeführt. Durch die Dialysierflüssigkeitskammer 47 des Dialysators 48 fließt dazu im Gegenstrom die Dialysierflüssigkeit.

Die Dialysierflüssigkeit wird in dem Disposable gemäß der Figuren 1 bis 3 bereitgestellt, das in die Aufnahmeeinheit 50 der Hämodialysevorrichtung eingelegt wird, die anhand der Figuren 4 bis 7 beschrieben ist.

Der Einlaß der Dialysierflüssigkeitskammer 47 des Dialysators 48 ist über eine Zuführleitung 51 mit dem Anschluß 8 des Anschlußteils 6 des Disposables D verbunden, während der Auslaß der Dialysierflüssigkeitskammer über eine Rückführleitung 52, in die eine Dialysierflüssigkeitspumpe 53 geschaltet ist, mit dem Anschluß 7 des Anschlußteils 6 des Disposables verbunden ist. Von der Rückführleitung 52 zweigt stromauf des Anschlußteils 6 eine Überlaufleitung 54, ab, die in einen Behälter 55 zur Aufnahme ultrafiltrierter Flüssigkeit mündet. Die Überlaufleitung 54 kann aber auch direkt an dem Disposable angeschlossen sein. Hierzu ist es aber erforderlich, daß das Disposable über einen Anschlußteil mit drei Anschlüssen verfügt. In die Überlaufleitung 54 ist eine regelbare Drossel 56 geschaltet, um die Ultrafiltrationsrate sinnvoll begrenzen zu können.

Die Schlauchanschlüsse an das Disposable können konventionelle Anschlußstücke sein. Entscheidend ist, daß das Disposable zur Inbetriebnahme der Dialysevorrichtung schnell an das Schlauchleitungssystem anschließbar bzw. zum Austausch des Disposables wieder abnehmbar ist. Es ist auch möglich, daß das Schlauchleitungssystem einstückig mit dem Disposable ausgebildet ist.

Zur Inbetriebnahme der Hämodialysevorrichtung wird das Disposable D in die Aufnahmeeinheit 50 eingelegt und mit einer für eine Dialysebehandlung ausreichenden Menge an Dialysierflüssigkeit befüllt. Anschließend wird die Hämodialysevorrichtung in Betrieb gesetzt. Die frische Dialysierflüssigkeit strömt dann aus der oberen Hälfte des Disposables in die Dialysierflüssigkeitskammer 47 und die verbrauchte Dialysierflüssigkeit wird wieder der unteren Hälfte des Disposables zugeführt, so daß eine Vermischung von frischer und verbrauchter Dialysierflüssigkeit vermieden wird. Hierbei wird die Tatsache ausgenutzt, daß die zurückgeleitete Dialysierflüssigkeit aufgrund der im äußeren Kreislauf eintretenden Wärmeverluste stets etwas kühler ist als die frische Dialysierflüssigkeit. Auch eine wärmeisolierende Ausbildung der Aufnahmeeinheit kann dazu beitragen, daß der radiale Temperaturgradient klein gehalten wird und somit Konvektionsströmungen vermieden werden. Die optimale Schichtung von frischer und verbrauchter Dialysierflüssigkeit wird durch die annähernd zylindrische Form des entfalteten Disposables noch unterstützt.

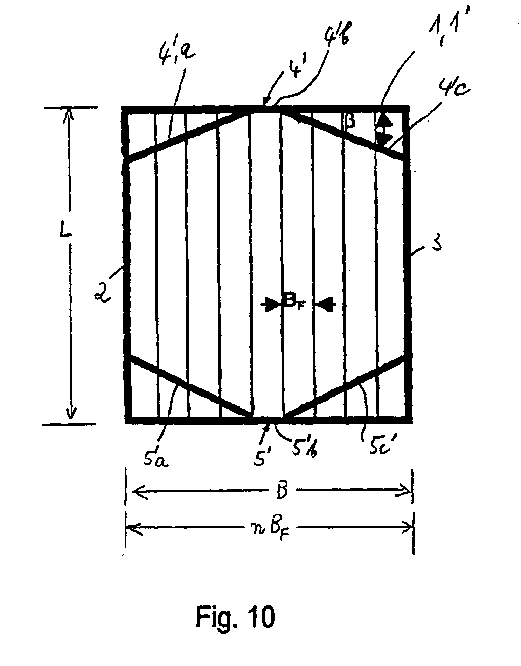

Fig. 10 zeigt ein weiteres Ausführungsbeispiel des Disposables in der Draufsicht, das für die Aufnahmeeinheit der Hämodialysevorrichtung von Fig. 9 bestimmt ist. Diese Ausführungsform unterscheidet sich von den oben beschriebenen Ausführungsbeispielen durch die Ausbildung der nach außen weisenden Schweißnähte. Die einander entsprechenden Teile sind mit den gleichen Bezugszeichen versehen.

Das Disposable weist zwei rechteckige Folien 1,1' mit der Länge L und der Breite B auf, die deckungsgleich übereinander liegen und an den Längsseiten mit Längschweißnähten 2,3 und an den Enden mit nach außen weisenden Schweißnähten 4',5' versiegelt sind. Der flachliegende Folienbeutel ist wieder parallel zu seiner Längsachse mehrfach in n gleich große Abschnitte harmonikaartig gefaltet, wobei die Breite B_{F} der Faltabschnitte sich aus dem Quotienten der Breite B der Flachfolien und der Anzahl der Faltabschnitte berechnet. Der Anschlußteil des Disposables ist in Figur 10 nicht dargestellt.

Die nach außen weisenden Schweißnähte bestehen jeweils aus drei geraden Abschnitten 4'a, 4'b, 4'c bzw. 5'a, 5'b, 5'c, die sich produktionstechnisch einfacher herstellen lassen, als bogenförmige Schweißnähte. Beide Schweißnähte 4', 5' weisen jeweils einen mittleren Geradenabschnitt 4'b, 5'b auf, dessen Endpunkte mit den äußeren Rändern der mittleren Faltung zusammenfallen. Zu beiden Seiten des mittleren Geradenabschnitts schließen sich äußere Geradenabschnitte 4'a, 4'c bzw. 5'a, 5'c an , die sich unter einem Winkel β bis zu den Längsschweißnähten 2,3 erstrecken. Der Winkel β ist so gewählt, daß die äußeren Geradenabschnitte den gekrümmten Verlauf der Bogenschweißnähte optimal approximieren. Er berechnet sich aus der mittleren Steigung der bogenförmigen Abschweißung.

## Patentansprüche

1. Disposable für eine Vorrichtung zur Durchführung einer medizinischen Behandlung unter Verwendung einer Flüssigkeit, insbesondere zur Bereitstellung von Dialysierflüssigkeit, wobei das Disposable ein Folienbeutel aus zwei übereinander liegenden Flachfolien (1, 1') oder eine Schlauchfolie mit Anschlüssen (7, 8) zum Zuführen und Abführen der Flüssigkeit ist, der bzw. die an dem in der Gebrauchslage oberen Ende mit einer ersten und an dem unteren Ende mit einer zweiten Schweißnaht (4, 5) verschlossen ist, **dadurch gekennzeichnet, dass** der Folienbeutel bzw. die Schlauchfolie parallel der in der Gebrauchslage vertikalen Längsachse harmonikaartig gefaltet ist, wobei die Faltungen des Folienbeutels bzw. der Schlauchfolie mit an den Folienenden angeordneten Verbindungselementen (13, 14) fixiert sind.

2. Disposable nach Anspruch 1, **dadurch gekennzeichnet, dass** die erste und zweite Schweißnaht (4,5) einen von einem geraden Verlauf abweichenden, zu dem in der Gebrauchslage oberen oder unteren Ende des Folienbeutels bzw. der Schlauchfolie weisenden Verlauf haben.

3. Disposable nach Anspruch 2, **dadurch gekennzeichnet, dass** die erste und zweite Schweißnaht (4, 5) bogenförmig sind.

4. Disposable nach Anspruch 2, **dadurch gekennzeichnet, dass** die erste und zweite Schweißnaht (4, 5) jeweils mehrere gerade Abschnitte aufweist.

5. Disposable nach einem der Ansprüche 1 bis 4, **dadurch gekennzeichnet, dass** an der ersten Schweißnaht (5) ein Anschlussteil (6) mit der Beutelfolie des Folienbeutels bzw. der Schlauchfolie verschweißt ist, der mindestens einen Anschluss (7) zum Zuführen von Flüssigkeit und einen Anschluss (8) zum Abführen von Flüssigkeit aufweist, wobei an dem Anschluss zum Zuführen von Flüssigkeit eine sich bis zu der zweiten Schweißnaht erstreckende Schlauchleitung (9) angeschlossen ist.

6. Disposable nach Anspruch 5, **dadurch gekennzeichnet, dass** an der zweiten Schweißnaht (4) ein Fixierungsstück (10) mit der Beutelfolie des Folienbeutels bzw. der Schlauchfolie verschweißt ist, an dem die Schlauchleitung (9) befestigt ist.

7. Disposable nach einem der Ansprüche 1 bis 6, **dadurch gekennzeichnet, dass** der Folienbeutel bzw. die Schlauchfolie aus einem Polyethylen-Basismaterial besteht, dass einseitig mit einer Polyamid-Siegelschicht versehen ist.

8. Verwendung eines Disposables nach einem der Ansprüche 1 bis 7 zur Bereitstellung von Dialysierflüssigkeit für eine Hämodialysevorrichtung oder einer Peritonealdialysevorrichtung.

## Claims

1. A disposable for a device for the performance of a medical treatment using a fluid, in particular for the preparation of dialysing fluid, whereby the disposable is a film bag consisting of two flat films (1, 1') lying one upon the other or a tubular film with connections (7, 8) for the supply and discharge of the fluid, said film bag or tubular film being closed at the - viewed in the position of use - upper end with a first and at the lower end with a second weld seam (4, 5), **characterised in that** the film bag or the tubular film is folded harmonica-like parallel to the - viewed in the position of use -vertical longitudinal axis, whereby the folds of the film bag or the tubular film are fixed with connection elements (13, 14) arranged at the film ends.

2. The disposable according to claim 1, **characterised in that** the first and second weld seam (4, 5) have a course diverging from a straight course and pointing towards the - viewed in the position of use - upper or lower end of the film bag or tubular film.

3. The disposable according to claim 2, **characterised in that** the first and second weld seam (4, 5) are arc-shaped.

4. The disposable according to claim 2, **characterised in that** the first and second weld seam (4, 5) each have a plurality of straight segments.

5. The disposable according to any one of claims 1 to 4, **characterised in that**, at the first weld seam (5), there is welded to the bag film of the film bag or the tubular film a connection piece (6), which has at least one connection (7) for the supply of fluid and a connection (8) for the discharge of fluid, whereby a tube line (9) extending up to the second weld seam is connected to the connection for the supply of fluid.

6. The disposable according to claim 5, **characterised in that**, at the second weld seam (4), there is welded to the bag film of the film bag or the tubular film a fixing piece (10), to which the tube line (9) is fixed.

7. The disposable according to any one of claims 1 to 6, **characterised in that** the film bag or the tubular film is made from a polyethylene base material, which is provided on one side with a polyamide sealing layer.

8. Use of a disposable according to any one of claims 1 to 7 for the preparation of dialysing fluid for a haemodialysis apparatus or a peritoneal dialysis apparatus.

## Revendications

1. Elément jetable pour un dispositif de mise en oeuvre d'un traitement médical en utilisant un liquide, en particulier pour la mise à disposition de liquide de dialyse, l'élément jetable étant un sac en feuille constitué par deux feuilles planes superposées (1, 1') ou une feuille tubulaire, avec des raccords (7, 8) pour l'entrée et la sortie du liquide, qui, en position d'utilisation, est fermé à l'extrémité supérieure par une première soudure et à l'extrémité inférieure par une seconde soudure (4, 5), **caractérisé en ce que** le sac en feuille ou la feuille tubulaire est plié en accordéon parallèlement à l'axe longitudinal vertical en position d'utilisation, et les plis du sac en feuille ou de la feuille tubulaire sont fixés aux éléments de fixation (13, 14) disposés à l'extrémité de la feuille.

2. Elément jetable selon la revendication 1, **caractérisé en ce que** la première et la seconde soudure (4,5) suivent un trajet qui s'écarte du trajet droit, trajet menant en position d'utilisation aux extrémités supérieure ou inférieure du sac en feuille ou de la feuille tubulaire.

3. Elément jetable selon la revendication 2, **caractérisé en ce que** la première et la seconde soudure (4, 5) se présentent sous forme courbée.

4. Elément jetable selon la revendication 2, **caractérisé en ce que** la première et la seconde soudure (4, 5) présentent chacune plusieurs segments droits.

5. Elément jetable selon l'une des revendications 1 à 4, **caractérisé en ce que**, sur la première soudure (5), une pièce de raccordement (6) est soudée à la feuille du sac de feuille ou la feuille tubulaire et présente au moins un raccord (7) pour l'entrée du liquide et un raccord (8) pour la sortie du liquide, au raccord pour l'entrée du liquide étant raccordé une conduite souple qui s'étend jusqu'à la seconde soudure.

6. Elément jetable selon la revendication 5, **caractérisé en ce que**, sur la seconde soudure (4), une pièce de fixation (10) est soudée avec la feuille du sac en feuille ou de la feuille tubulaire, laquelle est fixée à la conduite souple (9).

7. Elément jetable selon l'une des revendications 1 à 6, **caractérisé en ce que** le sac en feuille ou la- feuille tubulaire est en une matière à base de polyéthylène, qui est pourvue d'un côté d'une couche de revêtement de polyamide.

8. Utilisation d'un élément jetable selon l'une des revendications 1 à 7 pour la mise à disposition de liquide de dialyse pour un dispositif d'hémodialyse ou d'un dispositif de dialyse péritonéale.
